(19) Europäisches Patentamt
European Patent Office
Office européen des brevets

(11) Veröffentlichungsnummer : **0 014 999**
**B1**

(12) # EUROPÄISCHE PATENTSCHRIFT

(45) Veröffentlichungstag der Patentschrift : **28.04.82**

(51) Int. Cl.³ : **C 07 D265/30**, A 01 N 43/84// C07B19/00

(21) Anmeldenummer : **80100846.7**

(22) Anmeldetag : **21.02.80**
**Die Akte enthält technische Angaben, die nach dem Eingang der Anmeldung eingereicht wurden und die nicht in dieser Patentschrift enthalten sind.**

(54) **Optisch aktive Formen des 4-(3-(p-tert.-Butylphenyl)-2-methyl-propyl)-cis-2,6-dimethyl-morpholins und ihre Salze; Fungizide, die diese Verbindungen enthalten, und Verfahren zu ihrer Herstellung.**

(30) Priorität : **27.02.79 DE 2907614**

(43) Veröffentlichungstag der Anmeldung : **03.09.80 (Patentblatt 80/18)**

(45) Bekanntmachung des Hinweises auf die Patenterteilung : **28.04.82 Patentblatt 82/17**

(84) Benannte Vertragsstaaten : **AT BE CH DE FR GB IT LU NL SE**

(56) Entgegenhaltungen : **DE - A - 2 656 747**

(73) Patentinhaber : **BASF Aktiengesellschaft**
**Carl-Bosch-Strasse 38**
**D-6700 Ludwigshafen (DE)**

(72) Erfinder : **Himmele, Walter, Dr.**
**Eichenweg 14**
**D-6909 Walldorf (DE)**
Erfinder : **Pommer, Ernst-Heinrich, Dr.**
**Berliner Platz 7**
**D-6703 Limburgerhof (DE)**
Erfinder : **Siegel, Hardo, Dr.**
**Hans-Purrmann-Allee 25**
**D-6720 Speyer (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Optisch aktive Formen des 4-(3-(p-tert.-Butylphenyl)-2-methyl-propyl)-cis-2,6-dimethyl-morpholins und ihre Salze ; Fungizide, die diese Verbindungen enthalten, und Verfahren zu ihrer Herstellung

Die vorliegende Erfindung betrifft neue wertvolle optisch aktive Formen des 4-[3-(p-tert.-Butylphenyl)-2-methyl-propyl]-cis-2,6-dimethylmorpholins und ihre Salze mit guten fungiziden Wirkungen, Fungizide, die diese Verbindungen enthalten, und Verfahren zu ihrer Herstellung.

Es ist aus DE-A-26 56 747, 27 52 096 und 27 52 135 bekannt, daß 1-[3-(p-tert.-Butylphenyl)-2-methyl-propyl]-amine der Formel I

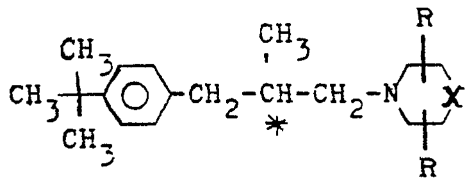

(I)

X = $CH_2$, O, S

R = $CH_3$

hervorragende fungizide Wirkungen gegenüber Getreidemehltau und Rostarten besitzen. Alle Verbindungen dieses Typs haben ein Chiralitätszentrum am C 2 der Propylkette (*). Eine besonders wirksame Verbindung ist das 1-[3-(p-tert.-Butylphenyl)-2-methylpropyl]-cis-3,5-dimethylmorpholin (II).

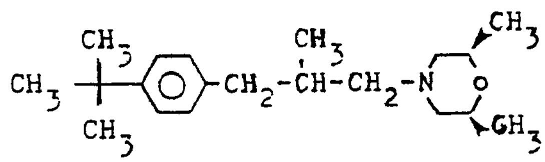

(II)

Es wurde nun gefunden, daß das (−)-Enantiomere des 4-[3-(p-tert.-Butylphenyl)-2-methylpropyl]-cis-2,6-dimethylmorpholins überraschend eine gute fungizide Wirkung hat, die der Wirkung des entsprechenden (+)-Enantiomeren überlegen ist. Das (−)-Enantiomere läßt sich beispiels-weise durch Umsetzung der Verbindung II mit (+)-Campfersulfonsäure in einem Verdünnungsmittel, anschließender Trennung der diastereomeren Salze durch fraktionierte Kristallisation und Umsetzung des das (−)-Enantiomere enthaltenden Salzes mit einer starken Base herstellen. Dieses Enantiomere wird bevorzugt.

In entsprechender Weise erhält man das (+)-Enantiomere aus der Verbindung II beispielsweise durch Umsetzung mit (−)-O,O'-Dibenzoyl-L-Weinsäure.

Es wurde ferner gefunden, daß das (+)-Enantiomere des 4-[3-(p-tert.-Butylphenyl)-2-methylpropyl]-cis-2,6-dimethylmorpholins überraschend eine andere fungizide Wirkung hat als das entsprechende (−)-Enantiomere.

Durch Röntgenstrukturanalyse wurde festgestellt, daß das (−)-Enantiomere S- und das (+)-Enantiomere R-Konfiguration besitzt.

Die vorliegende Erfindung betrifft auch die Salze der neuen Verbindungen, beispielsweise die Salze mit anorganischen oder organischen Säuren z.B. Salzsäure, Bromwasserstoffsäure, Jodwasserstoffsäure, Schwefelsäure, Phosphorsäure, Essigsäure Propionsäure, Adipinsäure, Maleinsäure, Fumarsäure, Benzoesäure außer Säuren, die für Pflanzen nicht verträglich sind. Die Erfindung umfaßt auch Salze mit optisch aktiven Säuren z.B. (+) und (−)-Mandelsäure, (+) und (−)-Weinsäure, L-Lysin, (+) und (−)-Äpfelsäure, L-Glutaminsäure, insbesondere die diastereomeren Salze. Solche Salze sind beispielsweise die in den Beispielen 1 und 2 beschriebenen Salze.

## Beispiel 1

Herstellung des (−)-4-[3-(p-tert.-Butylphenyl)-2-methylpropyl]-cis-2,6-dimethylmorpholins

303 g 4-[3-(p-tert.-Butylphenyl-2-methylpropyl]-cis-2,6-dimethylmorpholin und 250 g (+)-Campfer-10-sulfonsäure werden zusammen mit 500 ml Isopropanol auf ca. 80 °C erwärmt. Man läßt langsam abkühlen, saugt das entstehende Kristallisat ab (Fp 175-180 °C) und kristallisiert nochmals aus 500 ml Isopropanol um.

Man erhält 183 g campfersulfonsaures Salz Fp 191-192 °C, $[\alpha]_D^{20} = +29{,}3°$ (c = 1,1 Methanol).

Das Salz wird mit 30 %iger (Gew.-%) wässriger KOH versetzt, man trennt die organische Phase ab und destilliert. Ausbeute 85 g.

Kp 142 °C/0,4 mbar $[\alpha]_D^{20} = 4{,}8°$ (pur)

## Beispiel 2

Herstellung des (+)-4-[3-(p-tert.-Butylphenyl)-2-methylpropyl]-cis-2,6-dimethylmorpholins

68 g der Verbindung II und 85 g (−)-O,O′-Dibenzoyl-L-Weinsäure werden in 300 ml Isopropanol auf ca. 80 °C erhitzt.

Man verfährt wie in Beispiel 1 beschrieben und erhält 60 g dibenzoylweinsaures Salz $[\alpha]_D^{20}$ = 74,8° (c = 1,4, Methanol).

Aus dem Salz wird mit KOH wie in Beispiel 1 beschrieben das (+)-4-[3-(p-tert.-Butylphenyl)-2-methylpropyl]-cis-2,6-dimethylmorpholin in Freiheit gesetzt.

Kp 142 °C/0,4 mbar $[\alpha]_D^{20}$ = + 4,9° (pur)

Der erfindungsgemäße Wirkstoff und die entsprechenden Fungizide sind insbesondere geeignet zur Bekämpfung von Pflanzenkrankheiten, z.B. Erysiphe graminis (echter Mehltau) an Getreide, Erysiphe cichoriacearum (echter Mehltau) an Kürbisgewächsen, Podosphaera leucotricha an Äpfeln, Uncinula necator an Reben, Erysiphe polygoni an Bohnen. Sphaerotheca pannosa an Rosen, Microsphaera querci an Eichen, Botrytis cinerea an Erdbeeren, Reben, Mycosphaerella musicola an Bananen, Puccinia-Arten (Rostpilze) an Getreide, Uromyces appendiculatus und U. phaseoli an Bohnen, Hemuleia vastatrix an Kaffee und Rhizoctonia solani. Sie sind systematisch wirksam ; sie werden sowohl über die Wurzeln als auch über die Blätter aufgenommen und im Pflanzengewebe transportiert.

Bei der Anwendung des neuen Wirkstoffs zur Behandlung von Pflanzen gegen Pilzinfektionen liegen die Aufwandmengen zwischen 0,025 und 5 kg Wirkstoff/ha Fläche. Zum Oberflächenschutz von Bäumen oder Früchten kann der Wirkstoff auch in Verbindung mit Kunststoffdispersionen 0,25 %ig bis 5 %ig, bezogen auf das Gewicht der Dispersion, verwendet werden. Die Fungizide enthalten im allgemeinen zwischen 0,1 und 95 Gewichtsprozent Wirkstoff, vorzugsweise zwischen 0,5 und 90 %.

Der Wirkstoff kann mit anderen bekannten Fungiziden gemischt werden. In vielen Fällen erhält man dabei eine Vergrößerung des fungiziden Wirkungsspektrums; bei einer Anzahl von Fungizidmischungen in den Gewichtsverhältnissen 1 : 10 bis 10 : 1 treten auch synergistische Effekte auf, d.h., die fungizide Wirksamkeit des Kombinationsproduktes ist größer als die der addierten Wirksamkeiten der Einzelkomponenten. Fungizide, die mit den erfindungsgemäßen Verbindungen kombiniert werden können, sind beispielsweise :

Dithiocarbamate und deren Derivate, wie
- Zinkdimethyldithiocarbamat,
- Manganäthylenbisdithiocarbamat,
- Mangan-Zink-äthylendiamin-bis-dithiocarbamat,
- Zinkäthylenbisdithiocarbamat,
- Tetramethylthiuramdisulfid,
- Ammoniak-Komplex von Zink-(N,N-äthylen-bis-dithiocarbamat) und N,N′-Polyäthylen-bis-(thiocarbamoyl)-disulfid,
- Zink-(N,N′-propylen-bis-dithiocarbamat),
- Ammoniak-Komplex von Zink-(N,N′-propylen-bis-dithiocarbamat) und N,N′-Polypropylen-bis-(thiocarbamoyl)-disulfid ;

heterocyclische Verbindungen, wie
- N-Trichlormethylthio-tetrahydrophthalimid,
- N-Trichlormethylthio-phthalimid,
- N-(1,1,2,2-Tetrachloräthylthio)-tetrahydrophthalimid,
- 1-(Butylcarbamoyl)-2-benzimidazol-carbaminsäuremethylester,
- 2-Methyloxycarbonylamino-benzimidazol,
- 2,3-Dihydro-5-carboxanilido-6-methyl-1,4-oxathiin-4,4-dioxid,
- 2,3-Dihydro-5-carboxanilido-6-methyl-1,4-oxathiin,
- 5-Butyl-2-dimethylamino-4-hydroxy-6-methyl-pyrimidin,
- 1,2-Bis-(3-äthoxycarbonyl-2-thioureido)-benzol,
- 1,2-Bis-(3-methoxycarbonyl-2-thioureido)-benzol

und verschiedene andere Fungizide, wie
- Dodecylguanidinacetat,
- N-Dichlorfluormethylthio-N′,N′-dimethyl-N-phenyl-schwefelsäurediamid,
- 2,5-Dimethyl-furan-3-carbonsäureanilid,
- 2,5-Dimethyl-furan-3-carbonsäure-cyclohexylamid,
- 2-Jodbenzoesäureanilid,
- 2-Brombenzoesäureanilid,
- 3-Nitro-isophthalsäure-diisopropylester,
- 1-(1,2,4-Triazolyl-1-1′)-1-(4′-chlorphenoxy)-3,3-dimethyl-butan-2-on,
- 1-(1-Imidazolyl)-2-allyloxy-2-(2,4-dichlorphenyl)-äthan,
- Piperazin-1,4-diyl-bis-1-(2,2,2-trichloräthyl)-formamid,
- 2,4,5,6-Tetrachlor-isophthalonitril,
- 1,2-Dimethyl-3,5-diphenyl-pyrazoliniummethylsulfat.

Die Anwendung erfolgt z.B. in Form von direkt versprühbaren Lösungen, Pulvern, Suspensionen

oder Dispersionen, Emulsionen, Öldispersionen, Pasten, Stäubemitteln, Streumitteln, Granulaten durch Versprühen, Vernebeln, Verstäuben, Verstreuen, Beizen oder Gießen. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten ode Öldispersionen können die Substanzen als solche oder in einem Öl oder Lösungsmittel gelöst, mittels Netz-, Haft-, Dispergieroder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen : Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol-, Octylphenol-, Nonylphenol, Alkylphenolpolyglykoläther. Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Silikate, Talkum, Kaolin, Attaclay, Kalkstein, Kalk, Kreide, Talkum, Bolus, Löß, Ton, Dolomit, Diatemeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Zu den Mischungen oder Einzelwirkstoffen können Öle verschiedenen Typs, Herbizide, Fungizide, Nematozide, Insektizide, Bakterizide, Spurenelemente, Düngemittel, Antischaummittel (z.B. Silikone), Wachstumsregulatoren, Antidotmittel oder andere wirksame Verbindungen, zugesetzt werden.

## Beispiel 3

### Weizenmehltau

Blätter von in Töpfen gewachsenen Weizenkeimlingen der Sorte « Jubilar » werden mit wäßrigen Emulsionen aus 80 % (Gewichtsprozent) Wirkstoff und 20 % Emulgiermittel besprüht und nach dem Antrocknen des Spritzbelages mit Oidien (Sporen) des Weizenmehltaus (Erysiphe graminis var. tritici) bestäube. Die Versuchspflanzen werden anschließend im Gewächshaus bei Temperaturen zwischen 20 und 22 °C und 75 bis 80 % relativer Luftfeuchtigkeit aufgestellt. Nach 10 Tagen wird das Ausmaß der Mehltaupilzentwicklung ermittelt.

| Wirkstoff | Befall der Blätter nach Spritzungen mit x %iger Wirkstoffbrühe | | |
|---|---|---|---|
| x = | 0,012 | 0,006 | 0,003 |
| Wirkstoff gemäß Beispiel 1 | 0 | 0 | 0 |
| Wirkstoff gemäß Beispiel 2 | 0 | 2 | 2-3 |
| Verbindung II (DE-A-26 56 747) | 0 | 0 | 1-2 |
| Kontrolle (unbehandelt) | 5 | | |

Beispiel 4

Weizenbraunrost

Blätter von in Töpfen gewachsenen Weizenpflanzen werden mit Sporen des Weizenbraunrostes (Puccinia recondita) künstlich infiziert und 48 Stunden lang bei 20 bis 25 °C in einer wasserdampfgesättigten Kammer aufgestellt. Danaen werden die Pflanzen mit wäßrigen Spritzbrühen, die in dem Wasser gelöst oder emulgiert eine Mischung aus 80 % des zu prüfenden Wirkstoffes und 20 % Natriumligninsulfonat enthalten, besprüht und im Gewächshaus bei Temperaturen zwischen 20 und 22 °C und bei 75 bis 80 % relativer Luftfeuchtigkeit aufgestellt. Nach 10 Tagen wird das Ausmaß der Rostpilzentwicklung beurteilt.

| Wirkstoff | Befall der Blätter nach Spritzungen mit x %iger Wirkstoffbrühe x = 0,012 | 0,006 | 0,003 |
|---|---|---|---|
| Wirkstoff gemäß Beispiel 1 | 0 | 0 | 0 |
| Wirkstoff gemäß Beispiel 2 | 2 | 3 | 3 |
| Verbindung II (DE-A-26 56 747) | 0 | 0 | 2 |
| Kontrolle (unbehandelt) | 5 | | |

0 = kein Befall, abgestuft bis 5 = Totalbefall

Beispiel 5

Man vermischt 90 Gewichtsteile der Verbindung gemäß Beispiel 1 (A) mit 10 Gewichtsteilen N-Methyl-2-pyrrolidon und erhält eine Lösung, die zur Anwendung in Form kleinster Tropfen geeignet ist.

Beispiel 6

20 Gewichtsteile der Verbindung A werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel 7

20 Gewichtsteile der Verbindung A werden in einer Mischung gelöst, die aus 40 Gewichtsteilen Cyclohexanon, 30 Gewichtsteilen Isobutanol, 20 Gewichtsteilen des Anlagerungs produktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 8

20 Gewichtsteile der Verbindung A werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 9

20 Gewichtsteile des Wirkstoffs A werden mit 3 Gewichtsteilen des Natriumsalzes der Diisobutylnaphthalin-α-sulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 10

3 Gewichtsteile der Verbindung A werden mit 97 Gewichtsteilen feinteiligem Kaolin innig vermischt. Man erhält auf diese Weise ein Stäubemittel, das 3 Gewichtsprozent des Wirkstoffs enthält.

Beispiel 11

30 Gewichtsteile der Verbindung A werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 12

40 Gewichtsteile des Wirkstoffs A werden mit 10 Teilen Natriumsalz eines Phenolsulfonsäure-harnstoff-formaldehydkondensats, 2 Teilen Kieselgel und 48 Teilen Wasser innig vermischt. Man erhält eine stabile wäßrige Dispersion. Durch Verdünen mit 100 000 Gewichtsteilen Wasser erhält man eine wäßrige Dispersion, die 0,04 Gewichtsprozent Wirkstoff enthält.

Beispiel 13

20 Teile des Wirkstoffs A werden mit 2 Teilen Calciumsalz der Dodecylbenzolsulfonsäure, 8 Teilen Fettalkoholpolyglykoläther, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

**Ansprüche** (für die Vertragsstaaten : BE, CH, DE, FR, GB, IT, LU, NL, SE)

1. Als optisch aktive Formen der 4-[3-(p-tert.-Butylphenyl)-2-methylpropyl]-cis-2,6-dimethylmorpholins der Formel II

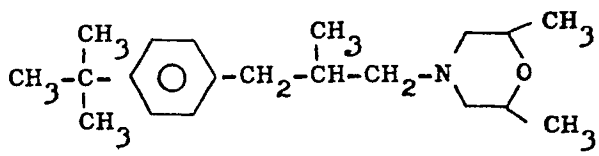

(II)

das (−)-4-[3-(p-tert.-Butylphenyl)-2-methylpropyl]-cis-2,6-dimethylmorpholin und seine Salze und das (+)-4-[3-(p-tert.-Butylphenyl)-2-methylpropyl]-cis-2,6-dimethylmorpholin und seine Salze.

2. Salz der (+)-Campfer-10-sulfonsäure mit (−)-4-[3-(p-tert.-Butylphenyl)-2-methylpropyl]-cis-2,6-dimethylmorpholin.

3. Salz der (−)-O,O′-Dibenzoyl-L-Weinsäure mit (+)-4-[3-(p-tert.-Butylphenyl]-2-methylpropyl]-cis-2,6-dimethylmorpholin.

4. Fungizid, enthaltend eine optisch aktive Verbindung gemäß Anspruch 1.

5. Fungizid, enthaltend einen festen oder flüssigen Trägerstoff und eine optisch aktive Verbindung gemäß Anspruch 1.

6. Verfahren zur Herstellung eines Fungizids, dadurch gekennzeichnet, daß man einen festen oder flüssigen Trägerstoff mit einer optisch aktiven Verbindung gemäß Anspruch 1 vermischt.

7. Verfahren zur Bekämpfung von Pilzen, dadurch gekennzeichnet, daß man die Pilze oder die vor Pilzbefall zu schützenden Gegenstände mit einer fungizid wirksamen Menge einer optisch aktiven Verbindung gemäß Anspruch 1 behandelt.

**Ansprüche** (für den Vertragsstaat AT)

1. Fungizid, enthaltend das (−)-4-[3-(p-tert.-Butylphenyl)-2-methylpropyl]-cis-2,6-dimethylmorpholin der Formel II

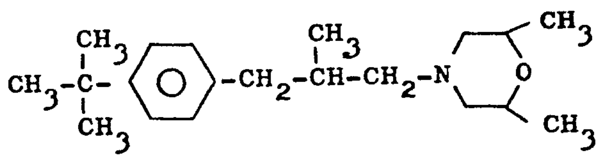

(II)

oder seine Salze oder das (+)-4-[3-(p-tert.-Butylphenyl)-2-methylpropyl]-cis-2,6-dimethylmorpholin der Formel II oder seine Salze.

2. Fungizid, enthaltend einen festen oder flüssigen Trägerstoff und eine der im Anspruch 1 definierten optisch aktiven Verbindungen.

3. Verfahren zur Bekämpfung von Pilzen, dadurch gekennzeichnet, daß man die Pilze oder die vor Pilzbefall zu schützenden Gegenstände mit einer fungizid wirksamen Menge einer der in Anspruch 1 definierten optisch aktiven Verbindungen behandelt.

**Claims** (for the contracting States : BE, CH, DE, FR, GB, IT, LU, NL, SE)

1. Optically active forms of 4-[3-(p-tert-butylphenyl)-2-methylpropyl]-cis-2,6-dimethylmorpholine of the formula II

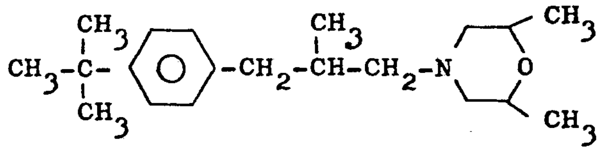
(II)

(−)-4-[3-(p-tert-butylphenyl)-2-methylpropyl]-cis-2,6-dimethylmorpholine and its salts, and (+)-4-[3-(p-tert-butylphenyl)-2-methylpropyl)-cis-2,6-dimethylmorpholine and its salts.

2. The salt of (+)-camphor-10-sulphonic acid with (−)-4-[3-(p-tert-butylphenyl)-2-methylpropyl]-cis-2,6-dimethylmorpholine.

3. The salt of (−)-O,O'-dibenzoyl-L-tartaric acid with (+)-4-[3-(p-tert-butylphenyl)-2-methylpropyl]-cis-2,6-dimethylmorpholine.

4. A fungicide containing an optically active compound as claimed in claim 1.

5. A fungicide containing a solid or liquid carrier and an optically active compound as claimed in claim 1.

6. A process for the production of a fungicide, characterized in that a solid or liquid carrier is mixed with an optically active compound as claimed in claim 1.

7. A process for combating fungi, characterized in that the fungi or the objects to be protected against fungus attack are treated with a fungicidally effective amount of an optically active compound as claimed in claim 1.

**Claims** (for contracting State AT)

1. A fungicide containing (−)-4-[3-(p-tert-butylphenyl)-2-methylpropyl]-cis-2,6-dimethylmorpholine of the formula II

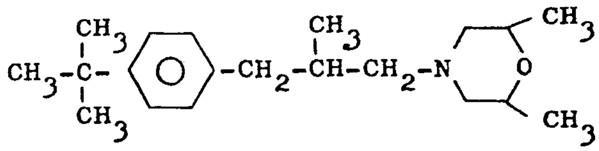
(II)

or its salts, or (+)-4-[3-(p-tert-butylphenyl)-2-methylpropyl]-cis-2,6-dimethylmorpholine of the formula II or its salts.

2. A fungicide containing a solid or liquid carrier and one of the optically active compounds defined in claim 1.

3. A process for combating fungi, characterized in that the fungi or the objects to be protected against fungus attack are treated with a fungicidally effective amount of one of the optically active compounds defined in claim 1.

**Revendications** (pour les Etats contractants : BE, CH, DE, FR, GB, IT, LU, NL, SE)

1. En tant que formes optiquement actives de la 4-[3-(p-tert.-butylphényl)-2-méthylpropyl]-cis-2,6-diméthylmorpholine de formule II

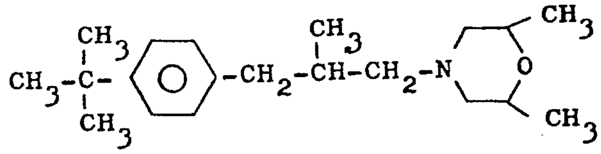

(II)

la (−)-4-[3-(p-tert.-butylphényl)-2-méthylpropyl]-cis-2,6-diméthylmorpholine et ses sels et la (+)-4-[3-(p-tert.-butylphényl)-2-méthylpropyl]-cis-2,6-diméthylmorpholine et ses sels.

2. Sel de l'acide (+)-camphre-10-sulfonique et de la (−)-4-[3-(p-tert.-butylphényl)-2-méthylpropyl]-cis-2,6-diméthylmorpholine.

3. Sel de l'acide (−)-O,O'-dibenzoyl-L-tartrique et de la (+)-4-[3-(p-tert.-butylphényl-2-méthylpropyl]-cis-2,6-diméthylmorpholine.

4. Fongicide contenant un composé optiquement actif selon la revendication 1.

5. Fongicide contenant un support solide ou liquide et un composé optiquement actif selon la revendication 1.

6. Procédé de préparation d'un fongicide, caractérisé par le fait qu'on mélange un support solide ou liquide avec un composé optiquement actif selon la revendication 1.

7. Procédé de lutte contre les champignons, caractérisé par le fait que l'on traite les champignons ou les objets à protéger contre les maladies des champignons avec une quantité efficace du point de vue fongicide d'un composé optiquement actif selon la revendication 1.

**Revendications** (pour l'Etat contractant AT)

1. Fongicide contenant la (−)-4-[3-(p-tert.-butylphényl)-2-méthylpropyl]-cis-2,6-diméthylmorpholine de formule II

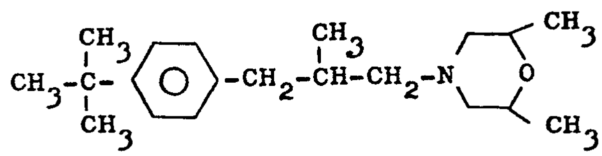

(II)

ou ses sels, ou la (+)-4-[3-(p-tert.-butylphényl)-2-méthylpropyl]-cis-2,6-diméthylmorpholine de formule II ou ses sels.

2. Fongicide contenant un support solide ou liquide et un composé optiquement actif défini dans la revendication 1.

3. Procédé de lutte contre les champignons, caractérisé par le fait que l'on traite les champignons ou les objets à protéger contre les maladies des champignons avec une quantité efficace du point de vue fongicide d'un composé optiquement actif selon la revendication 1.